(19) Europäisches Patentamt
European Patent Office
Office européen des brevets

(11) Veröffentlichungsnummer: **0 332 988**
**A2**

(12) # EUROPÄISCHE PATENTANMELDUNG

(21) Anmeldenummer: **89103968.7**

(22) Anmeldetag: **07.03.89**

(51) Int. Cl.[4]: **C07D 263/56 , C07C 103/78 , C08G 69/00**

(30) Priorität: **17.03.88 DE 3808914**

(43) Veröffentlichungstag der Anmeldung:
**20.09.89 Patentblatt 89/38**

(84) Benannte Vertragsstaaten:
**BE DE FR GB IT**

(71) Anmelder: **BAYER AG**
**D-5090 Leverkusen 1 Bayerwerk(DE)**

(72) Erfinder: **Köhler, Burkhard, Dr.**
**Virneburgstrasse 9**
**D-4150 Krefeld 11(DE)**
Erfinder: **Meyer, Rolf-Volker Dr.**
**Buchheimer Strasse 23**
**D-4150 Krefeld(DE)**

(54) **Neue 2-Aryl-benzoxazole und neue 2'-Hydroxybenzanilide.**

(57) Die Erfindung betrifft neue 2-Arylbenzoxazole, die aus o-Aminophenolen und 2,5-Dichlorbenzoylchlorid zugänglich sind und die als Zwischenprodukt bei deren Herstellung auftretenden 2′-Hydroxybenzanilide.

EP 0 332 988 A2

## Neue 2-Aryl-benzoxazole und neue 2′-Hydroxybenzanilide

Die Erfindung betrifft neue 2-Arylbenzoxazole, die aus o-Aminophenolen und 2,5-Dichlorbenzoylchlorid zugänglich sind und die als Zwischenprodukt bei deren Herstellung auftretenden 2′-Hydroxybenzanilide.

2-Aryl-benzoxazole sind bekannt (z.B. Robert C. Elderfield; Heterocyclic Compounds; John Wiley & Sons, London 1957). Sie lassen sich in bekannter Weise aus Aminophenolen gemäß folgendem Schema herstellen:

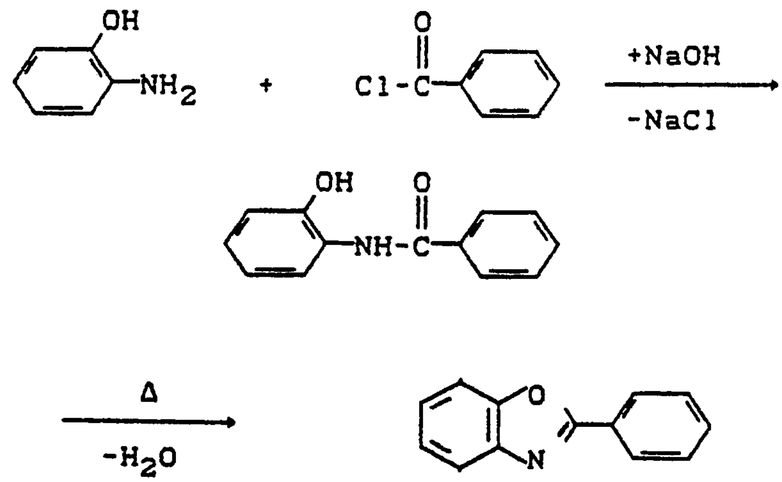


Ein weiterer möglicher Herstellungsweg kann auch von den entsprechenden Benzonitrilen ausgehen, die mit dem Hydrochlorid des o-Aminophenols erhitzt werden.

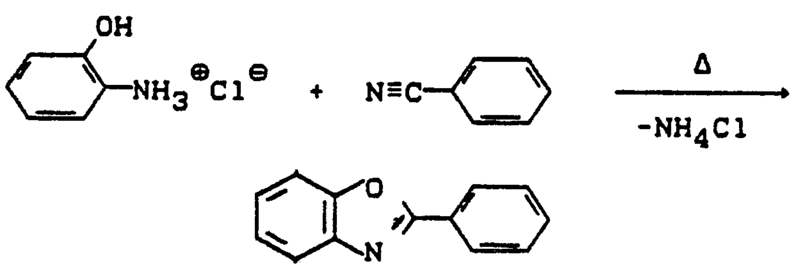


Ferner sind verschiedene Halogen-substituierte 2-Arylbenzoxazole bekannt, so z.B. das 6-Chlor-2-(4-chlorphenyl)benzoxazol und das 2-(2,4-Dichlorphenyl)-benzoxazol.

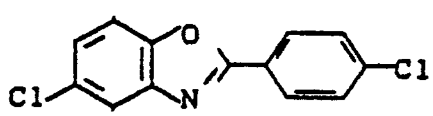

**6-Chlor-2-(4-chlorphenyl)-benzoxazol**

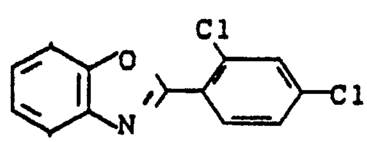

**2-(2,4-Dichlorphenyl)-benzoxazol**

Halogenierte 2-Arylbenzoxazole können als Monomere bei der Herstellung von Polymeren verwendet werden.

Es wurde nun gefunden, daß aus 2,5-Dichlorbenzoylchlorid und o-Aminophenolen neue 2′-Hydroxybenzanilide entstehen. Diese lassen sich dann thermisch zu neuen 2-(2,5-Dichlorphenyl)-benzoxazolen cyclisieren.

Gegenstand der Erfindung sind daher neue 2′-Hydroxybenzanilide der Formel (I)

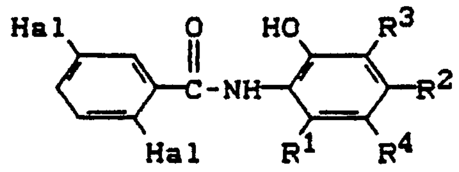 (I),

in welcher
$R^1$, $R^2$ und $R^3$ unabhängig voneinander Wasserstoff (H), einen $C_1$-$C_{22}$-Alkyl- bevorzugt einen Methyl-oder einen $C_6$-$C_{14}$-Aryl-, bevorzugt einen Phenylrest, besonders bevorzugt H bedeuten,
$R^4$ gleich $R^1$ bis $R^3$ ist oder Hal, vorzugsweise Chlor, bedeutet,
Hal unabhängig voneinander Fluor, Chlor, Brom oder Jod, bevorzugt Chlor bedeutet
und
die durch Cyclisierung aus diesen hergestellten 2-Arylbenzoxazole der Formel (II)

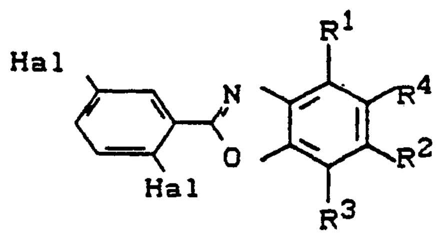 (II),

in welcher
$R^1$ bis $R^4$ und Hal die bei Formel I angegebene Bedeutung haben.

Als Beispiele für die erfindungsgemäßen 2-Arylbenzoxazole seien genannt: 2-(2,5-Dichlorphenyl)-benzoxazol, 6-Chlor-2-(2,5-dichlorphenyl)-benzoxazol usw.

Als Beispiele für die erfindungsgemäßen 2′-Hydroxybenzanilide seien genannt: 2,5-Dichlor-2′-hydroxybenzanilid, 2,5,5′-Trichlor-2′-hydroxybenzanilid usw.

Die Erfindung betrifft weiterhin ein Verfahren zur Herstellung der neuen 2-Aryl-benzoxazole, dadurch gekennzeichnet, daß Aminophenole der Formel (III)

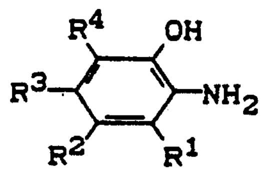 (III),

in welcher
$R^1$, $R^2$, $R^3$ und $R^4$ die bei Formel (I) angegebene Bedeutung haben,
in an sich bekannter Weise in Gegenwart einer Base in einem gegebenenfalls wäßrigen Verdünnungsmittel bei einer Temperatur von -25 bis 15° C, mit Benzoesäurehalogeniden der Formel (IV)

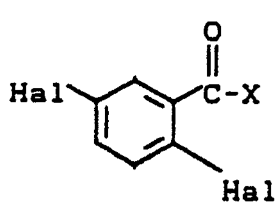 (IV),

in welcher
X für Halogen wie Chlor oder Brom und
Hal unabhängig voneinander für Fluor, Chlor, Brom und Jod steht,
zu 2′-Hydroxybenzamiden der Formel (I)

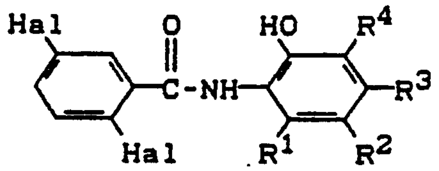

(I),

in welcher
Hal, $R^1$, $R^2$, $R^3$ und $R^4$ die oben angegebene Bedeutung haben,
umgesetzt werden und diese nach Isolierung in einer zweiten Umsetzung innerhalb von 2 bis 10 h bei einer Temperatur von 150 bis 250 °C, gegebenenfalls bei einem Druck von 1 bis $10^{-5}$ bar, zu den 2-Arylbenzoxazolen der Formel (II)

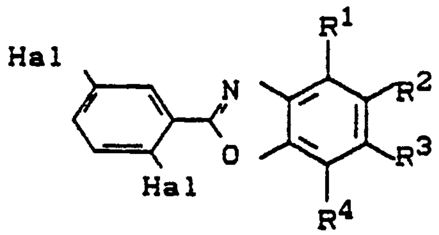

(II),

in welcher
Hal, $R^1$, $R^2$, $R^3$, $R^4$ die oben angegebene Bedeutung haben, cyclisiert werden.

Die Herstellung der Amide, die dann zu den 2-Arylbenzoxazolen cyclisiert werden, kann durch Aussetzung des entsprechenden Dihalogenbenzoylchlorids mit dem entsprechenden o-Aminophenol nach der allgemein bekannten Schotten-Baumann-Reaktion in einem wässrigen Medium, vorzugsweise in Wasser, in Gegenwart anorganischer Basen, z.B. NaOH, KOH, u.s.w. erfolgen. Diese Reaktion kann auch in organischen Solventien wie Methylenchlorid, Diethylether oder Toluol, in Gegenwart organischer Basen, z.B. Triethylamin, Pyridin oder N,N-Dimethylanilin, u.s.w., vorgenommen werden.

Als Beispiele für die verwendeten Aminophenole seien genannt: 2-Aminophenol, 2-Amino-4-chlorphenol, 2-Amino-3-methylphenol, 2-Amino-4-bromphenol, 2-Amino-6-phenylphenol, vorzugsweise 2-Amino-phenol und 2-Amino-4-chlorphenol usw.

Als Beispiele für die verwendeten Dihalogenbenzoylchloride seien genannt: 2,5-Dichlorbenzoylchlorid, 2,5-Dibrombenzoylchlorid, 2,5-Difluorbenzoylchlorid, bevorzugt 2,5-Dichlorbenzoylchlorid usw.

Die auf diese Weise erhaltenen Amide werden vorzugsweise ohne Verdünnungsmittel auf 150 °C-300 °C, vorzugsweise auf 190-220 °C erhitzt, wobei die Cyclisierung zum 2-Arylbenzoxazol erfolgt.

Die Cyclisierung der Amide kann in Gegenwart eines Verdünnungsmittels, z.B. wasserentziehender Mittel wie Acetanhydrid oder Polyphosphorsäure und/oder in Gegenwart von über 150 °C siedenden Solventien wie Dimethylformamid, Diethylenglykoldiemthylether oder N-Methylpyrrolidon vorgenommen werden. Die Reinigung der 2-Arylbenzoxazole kann durch Vakuumdestillation oder Umkristallisieren erfolgen.

Die erfindungsgemäßen 2-Arylbenzoxazole eignen sich als Hilfsmittel für Polymere und als Monomerbausteine für Polymere, insbesondere für die Herstellung von temperaturbeständigen Polykondensaten.

Die bei der Synthese der 2-Arylbenzoxazole als Zwischenprodukte auftretenden 2′-Hydroxybenzanilide können außer als Zwischenprodukte für 2-Arylbenzoxazole auch als Flammschutzmittel für Polymere und als Endgruppen für Polyamide eingesetzt werden.

## Beispiel 1

Man mischt 255 g (2,342 mol) 2-Aminophenol, 93,7 g (2,342 mol) NaOH und 320 ml Wasser und gibt unter Eiskühlung 490,7 g (2,432 mol) 2,5-Dichlorbenzoylchlorid so zu, daß die Temperatur nicht über 10 °C ansteigt. Dabei fällt 2-(2,5-Dichlorbenzoylamino)-phenol an. Man läßt auf Raumtemperatur kommen, rührt 30 Min. und saugt das 2,5-Dichlor-2′-hydroxy-benzanilid, das als Zwischenprodukt entsteht, ab und trocknet es im Vakuumtrockenschrank bei 80 °C und 20 Torr. Das Amid wird anschließend 6h auf 200 °C erhitzt, wobei das entstehende Reaktionswasser abdestilliert. Nach Destillation des Rückstandes erhält man 504,77 g (81,5 %) 2-(2,5-Dichlorphenyl)-benzoxazol vom Siedepunkt 179 °C/3 hPa und vom Schmelzpunkt 90 °C.

Beispiel 2

Man verfährt wie in Beispiel 1 beschrieben. Statt 255 g 2-Aminophenol werden 335,7 g 2-Amino-4-chlorphenol eingesetzt. Als Zwischenprodukt wird 2,5,5′-Trichlor-2′-hydroxy-benzanilid isoliert. Man erhält nach Destillation 502,3 g (71,7 %) 6-Chlor-2-(2,5-dichlorphenyl)-benzoxazol vom Siedepunkt 243 °C/1,7 hPa und vom Schmelzpunkt 132 °C.

Die Elementaranalysen (C, H, N) der Substanzen aus Beispiel 1-2 stimmen mit den berechneten Werten innerhalb einer Fehlergrenze von 0,3 % überein.

**Ansprüche**

1. 2′-Hydroxy-benzanilide der Formel (I)

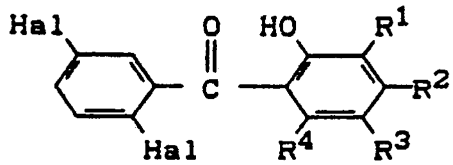

(I),

in welcher
Hal, $R^1$, $R^2$ und $R^3$ unabhängig voneinander Wasserstoff, H, einen $C_1$-$C_{22}$-Alkyl- bevorzugt einen Methyl- oder einen $C_6$-$C_{14}$-Aryl-, bevorzugt einen Phenylrest, besonders bevorzugt H,
$R^4$ gleich $R^1$ bis $R^3$ ist oder Hal, vorzugsweise Chlor bedeutet,
Hal unabhängig voneinander Fluor, Chlor, Brom oder Jod, bevorzugt Chlor bedeuten.

2. 2-Aryl-benzoxazole der Formel (II)

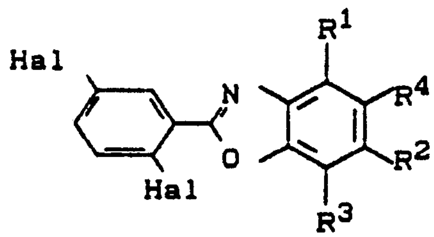

(II),

in welcher
$R^1$ bis $R^4$ und Hal die unter Anspruch 1 aufgeführte Bedeutung haben.

3. Verfahren zur Herstellung der 2-Aryl-benzoxazole nach Anspruch 1, dadurch gekennzeichnet, daß Aminophenole der Formel (III)

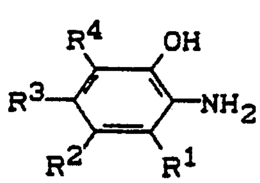

(III),

in welcher
$R^1$, $R^2$, $R^3$ und $R^4$ die in Anspruch 1 angegebene Bedeutung haben,
in an sich bekannter Weise in Gegenwart einer Base in einem gegebenenfalls wäßrigen Verdünnungsmittel bei einer Temperatur von -25 bis 15 °C, mit Benzoesäurehalogeniden der Formel (IV)

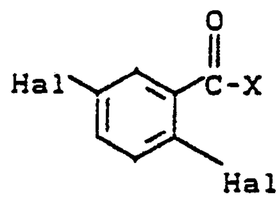

(IV),

in welcher
X für Halogen wie Chlor oder Brom und
Hal unabhängig voneinander für Fluor, Chlor, Brom und Jod steht,
zu 2′-Hydroxybenzamiden der Formel (I)

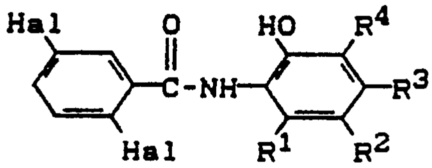

(I),

in welcher
Hal, $R^1$, $R^2$, $R^3$ und $R^4$ die in Anspruch 1 angegebene Bedeutung haben,
umgesetzt werden und diese nach Isolierung in einer zweiten Umsetzung innerhalb von 2 bis 10 h bei einer Temperatur von 150 bis 250 °C, gegebenenfalls in einem Verdünnungsmittel und gegebenenfalls bei einem Druck von 1 bis $10^{-5}$ bar, zu den 2-Arylbenzoxazolen der Formel (II)

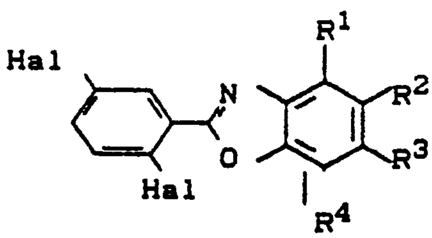

(II),

in welcher
Hal, $R^1$, $R^2$, $R^3$ und $R^4$ die in Anspruch 1 angegebene Bedeutung haben,
cyclisiert werden.

4. Verwendung von 2-Aryl-benzoxazolen nach Anspruch 1 als Monomere bei der Herstellung von Kunststoffen.